# EUROPEAN PATENT SPECIFICATION

(11) **EP 2 269 586 B1**
(45) Date of publication and mention of the grant of the patent: **21.09.2011**
(21) Application number: 09008618.2
(22) Date of filing: 01.07.2009
(51) Int. Cl.: A61K 9/20, A61K 9/28

(54) **Pharmaceutical composition comprising desloratadine**
Pharmazeutische Zusammensetzung mit Desloratadin
Composition pharmaceutique comprenant du desloratadine

(43) Date of publication of application: 05.01.2011
(73) Proprietor: Alfred E. Tiefenbacher (GmbH & Co. KG), 22767 Hamburg (DE)
(72) Inventor: Yogananda, Chaitanya, Gujjar, Shimoga 577 202 Karnataka (IN); Boddu, Srinath, Karimnagar 505001 Andhra Pradesh (IN); Rallabandi, Bala, Ramesha, Chary, Hyderabad 500 072 Andhra Pradesh (IN); Pasahn, Manohar, Lal, Thane 400 602 Maharashtra (IN); Fitzner, Ansgar, 20253 Hamburg (DE)
(74) Representative: Feldmann, Ute

(56) References cited:
- WO-A-2004/066984
- US-A1- 2008 118 555

## Description

### Technical Field:

The present invention relates to a pharmaceutical composition comprising desloratadine or a pharmaceutically acceptable salt thereof, use of the inventive pharmaceutical composition as well as a process of producing the inventive composition.

### Background of the invention:

EP 0 152 897 B1 discloses descarbonylethoxyloratadine with the chemical name 8-chloro-6,11-dihydro-11-(4-piperdinylidene)-5H-benzo[5,6]cyclohepta[1,2-b]pyridine (further referred to as "desloratadine", or "DCL") as a metabolic derivative of loratadine and a long-acting tricyclic histamine antagonist with selective H1-receptor histamine antagonist activity. Desloratadine is indicated for the relief of the nasal and non-nasal symptoms of allergic rhinitis (seasonal and perennial) as well as for the symptomatic relief of pruritus, reduction in the number and size of hives, in patients with urticaria.

WO 99/01450 A1 discloses several polymorphic forms of desloratadine, such as polymorph forms 1 and 2.

WO 96/20708 A1 discloses methods and compositions for treating allergic rhinitis and other disorders, such as allergic asthma, retinopathy and small vessel disorders associated with diabetes mellitus, cough, cold, cold-like and/or flu symptoms and the discomfort, pain, headache, fever, and general malaise associated therewith in human using as active ingredient desloratadine alone or in combination with non-steroidal anti-inflammatory agents or other non-narcotic analgesics.

WO 00/02560, however, describes that during the development of pharmaceutical compositions comprising desloratadine it has been discovered that desloratadine was found to discolor when stored at 75 % relative humidity ("RH") and a temperature of 40°C (so called "accelerated storage conditions" according to the International Conference on Harmonization (ICH), which imitate the conditions of long term storage). This color instability in the active ingredient was found to be apparently due to a very minute amount of a degradation product of desloratadine, namely N-Formyldesloratadine, caused by the presence of a wide variety of excipients commonly used in solid, especially tablet formulations, which are able to undergo Maillard reaction in presence of desloratadine. Excipients found to be unsuitable include acidic excipients including, but not limited to stearic acid, povidone, crospovidone, and other excipients having a pH in water less than 7, as well as other excipients such as lactose, lactose monohydrate, sodium benzoate and glyceryl behenate. Such discolored pharmaceutical compositions do not fulfill the requirement of stability and accordingly, the following approaches have been undertaken to overcome the drawback of degradation of desloratadine in solid pharmaceutical compositions:

WO 00/02560 teaches to combine desloratadine with a carrier medium comprising dibasic calcium phosphate and microcrystalline cellulose - in the absence of prior art excipients such as stearic acid or lactose - to provide stable pharmaceutical compositions.

In contrast thereto, EP 0 969 836 B1 discloses lactose-free, non-hygroscopic and anhydrous pharmaceutical compositions of desloratadine. Lactose-free - in the sense of EP 0 969 836 B1 - means that the amount of reactive excipient(s), if any, should be present in an amount insufficient to interact with desloratadine, *i.e.*, should be less than about 20 wt.-%, preferably less than 10 wt.-% and even more preferably less than 1 wt.-%. In case lactose is present in the pharmaceutical composition, EP 0 969 836 B1 teaches to use alpha-lactose monohydrate. As a second embodiment EP 0 969 836 B1 teaches to use large particles of desloratadine with a particle size distribution, wherein 40 wt.-% of desloratadine have a size of 250 µm or larger.

Other methods to reduce the decomposition of desloratadine in pharmaceutical compositions include adding an anti oxidant, preferably inorganic or organic sulfur (CN 1552324 A), an amino acid (EP 1 728 513 A2) or cyclodextrin (WO 2007/096733 A2) to the pharmaceutical composition or provide desloratadine in molecular admixture with a pharmaceutically acceptable polymer (WO 2008/138563 A1).

WO 2004/066984 A2 discloses an improved taste masking pharmaceutical composition comprising as a preferred active ingredient desloratadine and US 08/0118555 discloses a stable pharmaceutical composition containing desloratadine.

The above attempts to overcome the decomposition of desloratadine in solid pharmaceutical compositions in dosage form of a tablet show, that although lactose undergoes the decomposition reaction with desloratadine, it is, due to its characteristics, still preferred as filler material in the manufacture of a solid pharmaceutical composition in dosage form of a tablet comprising desloratadine.

Accordingly, there is still a need for providing a stable solid pharmaceutical composition in dosage form of a tablet comprising desloratadine and lactose, wherein the amount of impurities after storage, in particular of decomposition products of desloratadine in the presence of lactose is reduced, preferably without adding specific pharmaceutically acceptable excipients and/or adjuvants as set out above.

### Brief description of the invention:

The problem of the present invention is solved by the subjects of the independent claims. Advantages (preferred embodiments) are set out in the detailed description hereinafter as well as in the dependent claims.

Accordingly, a first aspect of the present invention relates to a solid pharmaceutical composition in dosage form of a tablet comprising or consisting of desloratadine or a pharmaceutically acceptable salt thereof and one or more pharmaceutically acceptable excipients and/or adjuvants including lactose, characterized in that a) desloratadine or the pharmaceutically acceptable salt thereof is present in a therapeutically effective amount and that b) the lactose is present as anhydrous lactose in an amount of 22 to 78 wt.-% based on the total weight of the solid pharmaceutical composition and that the tablet is obtainable by a process comprising the following steps:
a. providing desloratadine or a pharmaceutically acceptable salt thereof and one or more pharmaceutically acceptable excipients and/or adjuvants including anhydrous lactose,
b. blending the components provided in step a),
c. optional compacting the blended components of step b), and
d. compression of the blended components of step b) or compacts of step c) to form the solid pharmaceutical composition in dosage form of a tablet,
with the proviso, that the process steps a) through d) are carried out under dry conditions.

A second aspect of the present invention relates to a process of production of a solid pharmaceutical composition in dosage form of a tablet according to the present invention, characterized in that the process comprises or consists of the following steps:
a. providing desloratadine or a pharmaceutically acceptable salt thereof and one or more pharmaceutically acceptable excipients and/or adjuvants including anhydrous lactose,
b. blending the components provided in step a),
c. optional compacting the blended components of step b), and
d. compression of the blended components of step b) or compacts of step c) to form the solid pharmaceutical composition in dosage form of a tablet, characterized in that a) desloratadine or the pharmaceutically acceptable salt thereof is present in a therapeutically effective amount and that b) the lactose is present as anhydrous lactose in an amount of 22 to 78 wt.-% based on the total weight of the solid pharmaceutical composition in dosage form of a tablet,
with the proviso, that the process steps a) through d) are carried out under dry conditions.

A third aspect of the present invention relates to the use of anhydrous lactose in the production of a solid pharmaceutical composition in dosage form of a tablet according to the present invention, wherein the amount of anhydrous lactose is in the range of 22 to 78 wt.% based on the total weight of the solid pharmaceutical composition in dosage form of a tablet.

A fourth aspect of the present invention relates to the use of a solid pharmaceutical composition in dosage form of a tablet according of the present invention, wherein the solid pharmaceutical composition in dosage form of a tablet is used in the manufacture of a medicament for the treatment or prophylaxis of histamine-induced disorders.

The aspects of the present invention as set out hereinbefore can also comprise, if reasonable to a person skilled in the art, any possible combination of the preferred embodiments as set out in the dependent claims or disclosed in the following detailed description.

### Detailed description of the invention:

It has surprisingly been found by the present inventors, that by using anhydrous lactose in the amount of 22 to 78 wt.-% based on the total weight of a pharmaceutical composition in dosage form of a tablet comprising desloratadine obtainable according to the inventive process, the amount of impurities, in particular of decomposition products of desloratadine, after storage under accelerated storage conditions of 40°C and 75 % relative humidity (RH) for 1 to 3 months (accelerated storage conditions, ICH guideline Q 1 A (R2) "Stability Testing of new Drug Substances and Products") is lower than when using lactose monohydrate and, thus, stable solid pharmaceutical compositions in dosage form of a tablet can be provided with total impurity of not more than 1.0 wt.-%. Accordingly, the inventive solid pharmaceutical composition can use lactose as the preferred filler for compression without the need of further adding specific pharmaceutically acceptable excipients and/or adjuvants to inhibit the decomposition reaction between lactose and desloratadine.

In the context of the present invention the inventive pharmaceutical compositions is present in dosage form of a tablet. To further inhibit degradation of desloratadine, preferably the inventive pharmaceutical composition reduces interaction with oxygen and/or moisture in the air, preferably by using a suitable coating. Accordingly, the most preferred dosage forms of the inventive compositions are coated tablets.

In context of the present invention desloratadine can be used as the free base or in form of a pharmaceutically acceptable salt (also commonly referred to as desloratadine), preferably in form of the free base. Furthermore, desloratadine can be used in either polymorphic forms 1 or 2 or as a mixture of polymorphic forms 1 and 2. To further reduce the decomposition of desloratadine, preferably anhydrous or low moisture desloratadine is used in the manufacture of inventive compositions. Furthermore, the particle size distribution of desloratadine in the inventive composition is preferably such, that 60 wt.-% of desloratadine particles have a particle size of less than 250 µm (D₆₀ < 250 µm), more preferably D₉₀ < 45 µm and/or D₁₀₀ < 110 µm. Desloratadine is present in the inventive pharmaceutical composition in a therapeutically effective amount, preferably in the range of 1 to 15 wt.-%, more preferred 3 to 10 wt.-% based on the total weight of the inventive composition in dosage form of a tablet or- in case the inventive composition is present as a coated tablet - based on the total weight of the uncoated tablet (core of the coated tablet). In a most preferred embodiment of the present invention, the amount of desloratadine in one inventive dosage form is 2.5 mg, 5.0 mg, 7.5 mg or 10.0 mg calculated on the free base.

In the context of the present invention anhydrous lactose is used in the amount of 22 to 78 wt.-%, preferably 22 to 30 wt.-%, 45 to 55 wt.-% or 70 to 78 wt.-%, more preferably 25, 50 or 75 wt.-% based on the total weight of the inventive composition in dosage form of a tablet or - in case the inventive composition is present as a coated tablet - based on the total weight of the uncoated tablet (core of the coated tablet). Anhydrous lactose in the sense of the present invention shall comply with the requirements of the relevant monographs, in particular it shall comprise less than 1.0 %, preferably less than 0.5 wt.-% of water based on the European Pharmacopoeia monograph.

In addition or alternatively to the preferred embodiments as set out hereinbefore, the inventive composition can contain in addition to anhydrous lactose one, two, three, four, five or more further pharmaceutically acceptable excipients and/or adjuvants, preferably selected from the group of binders, disintegrants, flavoring agents, glidants, solubilizers, lubricants, coating polymers and aids and/or encapsulating material. Preferably, the further pharmaceutically acceptable excipients are suitable for the inventive production process, wherein the direct compression (steps a), b) and d)) or compaction-compression (steps a) through d)) procedure is carried out under dry conditions.

In a preferred embodiment one or more of the following substances, which act as binders and/or disintegrants are used: microcrystalline cellulose, maize starch, povidone, hydroxypropyl cellulose, copovidone, pregelatinized starch, dibasic calcium phosphate, sugar, low substituted hydroxypropylcellulose, mannitol, crospovidone, sodium starch glycolate, croscarmellose sodium or mixtures thereof, more preferred are microcrystalline cellulose, low substituted hydroxypropylcellulose, mannitol, the combination of microcrystalline cellulose and low substituted hydroxypropylcellulose, or the combination of microcrystalline cellulose and crospovidone. Still further preferred are inventive compositions, wherein
a. microcrystalline cellulose is in the range of 30 to 70 wt.-%, preferably 40 to 66 wt.-%,
b. low substituted hydroxypropylcellulose is in the range of 10 to 70 wt.-%, preferably 15 to 66 wt.-%,
c. mannitol is in the range of 40 to 70 wt.-%, preferably 50 to 66 wt.-%,
d. microcrystalline cellulose is in the range of 60 to 65 wt.-%, preferably 63 wt.-% and low substituted hydroxypropyl cellulose is in the range of 1 to 7 wt.-%, preferably 5 wt.-%, or
e. microcrystalline cellulose is in the range of 10 to 70 wt.-%, preferably 14 to 65 wt.-% and crospovidone is in the range of 1 to 5 wt.-%, preferably 2 to 3 wt.-%,
respectively based on the total weight of the inventive composition in dosage form of a tablet or - in case the inventive composition is present as a coated tablet - based on the total weight of the uncoated tablet (core of the coated tablet).

In addition or alternatively to the preferred embodiments as set out hereinbefore, the inventive composition comprises or consists of one or more substances, which act as lubricants, preferably fatty acids or fatty acid derivatives, such as alkali and earth alkali salts of stearic, lauric and/or palmitic acid, more preferred calcium stearate, magnesium stearate, sodium stearyl fumarate, sodium lauryl sulfate, talc, hydrogenated vegetable oil, zinc stearate, or mixtures thereof. Most preferably magnesium stearate is comprised in the inventive composition as the or one of the lubricants. In general, the amount of lubricants, preferably magnesium stearate is present in the range of 0 to 5 wt.-%, preferably, 1 to 4 wt.-% and most preferred 1 to 2 wt.-% based on the total weight of the inventive composition in dosage form of a tablet or - in case the inventive composition is present as a coated tablet - based on the total weight of the uncoated tablet (core of the coated tablet).

In addition or alternatively to the preferred embodiments as set out hereinbefore, the inventive solid pharmaceutical composition additionally comprises or consists of a pharmaceutically acceptable coating. Suitable inert coating agents and methods for coating particles or granules are well known in the art. Typically, inert coating agents comprise an inert film-forming agent dispersed in a suitable solvent, and may further comprise other pharmaceutically acceptable adjuvants, such as colorants and/or plasticizers. Preferably, the coating of the inventive composition comprises or consists of one or more of the following film forming components: hydroxypropyl cellulose, hydroxypropylmethyl cellulose, ethyl cellulose, polyvinyl alcohol, polyethylene glycol-polyvinyl alcohol graftpolymer or mixtures thereof. The ratio of hydroxypropyl cellulose : hydroxypropylmethyl cellulose is preferably in the range from 1:10 to 10:1, more preferably 1:1. The ratio of hydroxypropyl cellulose : hydroxypropylmethyl cellulose : ethyl cellulose is preferably in the range of 5:5:1 to 1:1:8. As colorants aluminium lakes, such as indigo carmine aluminium lake, and/or iron oxides, such as red iron oxide, and/or titanium dioxide are preferably used in suitable amounts.

In addition or alternatively to the preferred embodiments as set out hereinbefore, the inventive composition further comprises or consists of a therapeutically effective amount of one or more additional active ingredients. Suitable additional active ingredients include pharmaceutically acceptable salts, solvates, enantiomers or mixtures thereof, preferably selected from the group comprising or consisting of decongestants, preferably sympathomimetic nasal decongestants, *e.g.*, pseudoephedrine, or parasympatholytics, *e.g.*, oxybutynin; expectorants/mucolytics, *e.g.*, ambroxol; analgesics, opioid analgesics or non-narcotic analgesics; anti-inflammatory drugs, preferably anti-inflammatory non-steroidal drugs (NSAID) or COX-2 inhibitors; anti-tussives; bronchodilators; corticosteroids, *e.g.*, mometasone; leukotriene receptor antagonists, *e.g.*, montelukast; prostaglandin D₂ antagonists and/or neurokinin receptor antagonists.

In case the inventive composition comprises or consists of a therapeutically effective amount of one or more additional active ingredients, the inventive composition is preferably present as a multilayer, preferably bilayer tablet, wherein the therapeutically effective amount of desloratadine is present in a first layer and the or one of the additional active ingredients are present in one or more additional layers, preferably in one additional (second) layer.

In case the inventive composition is present as a coated tablet, the therapeutically effective amount of desloratadine is preferably present in the uncoated core of the tablet and the or one of the additional active ingredients is/are present in the coating of the tablet.

In addition or alternatively to the preferred embodiments as set out hereinbefore, the inventive composition does not comprise or consist of an anti oxidant, preferably in form of inorganic or organic sulfur (such anti oxidants as disclosed in CN 1552324 A) , an amino acid, preferably arginine, cystein, tyrosine, histidine, lysine and/or tryptophan (such amino acids as disclosed in EP 1 728 513 A2), dextrin (such cyclodextrins as disclosed in WO 2007/096733 42) , desloratadine in molecular admixture with a pharmaceutically acceptable polymer (such desloratadine - polymer molecular admixture as disclosed in WO 2008/138563 A1) triglycerides, wax and/or polyethylene glycol (PEG) (such triglycerides, wax and/or polyethylene glycol as disclosed in WO 2007/140987) . Furthermore in case water insoluble starch products (such starch products as disclosed in US 08/118555) are contained in the inventive composition one or more basic salts are also comprised.

With respect to the second aspect of the present invention, namely the inventive process of production of a solid pharmaceutical composition in dosage form of a tablet according to the present invention, the process steps are readily known in the art and can be carried out by persons skilled in the art. The process steps a) through d) are to be carried out under dry conditions, which mean in the context of the present invention that no solvent, in particular water shall be added. Preferably, the moisture and humidity conditions are such, that inventive solid pharmaceutical compositions are produced by the inventive process, which comprise or consists of an amount of unbound water of less than 5 wt.-%, preferably less than 3.5 wt.-%, more preferably less than 2 wt.-% based on the total weight of the inventive composition, whereby the unbound water is measured by the loss of drying method, wherein the composition is analysed at 105°C until a constant value is obtained for 1 to 2 minutes using a Mettler Toledo moisture analyzer. With respect to preferred embodiments of desloratadine, anhydrous lactose and the further pharmaceutically acceptable excipients and/or adjuvants suitable for the inventive production process of the second aspect of the present invention, we refer to the description thereof hereinbefore in connection with the inventive solid pharmaceutical composition of the first aspect of the present invention.

In addition or alternatively to the preferred embodiments as set out hereinbefore, desloratadine and one or more pharmaceutically acceptable excipients and/or adjuvants including anhydrous lactose are preferably provided in process step a) of the inventive process after being sifted, preferably 0.2 to 1 mm.

In addition or alternatively to the preferred embodiments as set out hereinbefore, the compaction in process step c) of the inventive process is preferably carried out by dry granulation technique readily known to a person skilled in the art. Preferably roller compactors, Fitzpatrick roller compactor mills and tabletting machines can be used. More preferably, the compacts produced in step c) are milled prior to carrying out step d). In a further preferred embodiment the (milled) compacts in step c) are sifted, preferably # 30 mesh (= 0.6 mm), and/or are lubricated with (pre sifted, prefera bly # 40 mesh (= 0.4 mm)) lubricants, preferably magnesium stearate, prior to compression in step d).

In accordance with process step d) of the inventive process the compression can be carried out by methods already known to a person skilled in the art, preferably by any available tablet compression method.

In addition or alternatively to the preferred embodiments as set out hereinbefore, the compressed solid pharmaceutical composition of step d) in dosage form of a tablet is in a subsequent step e) coated with one or more pharmaceutically acceptable excipients and/or adjuvants. Suitable methods are already known in the art and suitable pharmaceutically acceptable excipients and/or adjuvants have been already described with respect to the first aspect of the present invention.

As a result the present invention relates to a solid pharmaceutical composition in dosage form of a tablet comprising or consisting of desloratadine or a pharmaceutically acceptable salt thereof and one or more pharmaceutically acceptable excipients and/or adjuvants including lactose obtainable by the inventive process as described hereinbefore, characterized in that a) desloratadine or a pharmaceutically acceptable salt thereof is present in a therapeutically effective amount and that b) the lactose is present as anhydrous lactose in an amount of 22 to 78 wt.-% based on the total weight of the solid pharmaceutical composition in dosage form of a tablet. With respect to the preferred embodiments we refer to the description thereof hereinbefore in connection with the inventive solid pharmaceutical composition of the first aspect of the present invention.

With respect to the third aspect of the present invention preferred embodiments of suitable anhydrous lactose have been described hereinbefore in connection with the first aspect of the present invention.

With respect to the fourth aspect of the present invention the medicament is preferably used in the treatment or prophylaxis of allergic rhinitis, allergic asthma, urticaria, symptomatic dermographism, diabetic retinopathy and other small vessel disorders associated with diabetes mellitus, as well as cough, cold, cold-like and/or flu symptoms and the discomfort, pain, headache, fever, and general malaise associated therewith.

The present invention is explained further with the aid of the following non-limiting examples, illustrating the parameters of and compositions employed within the present invention. Unless stated otherwise, all data, in particular percentages, parts and ratios are by weight.

### Examples:

### Part A: Process of production of pharmaceutical compositions

The following procedure illustrates the production procedures of comparative composition (C1) and inventive pharmaceutical compositions (F1 to F13) as (coated) tablet formulations:

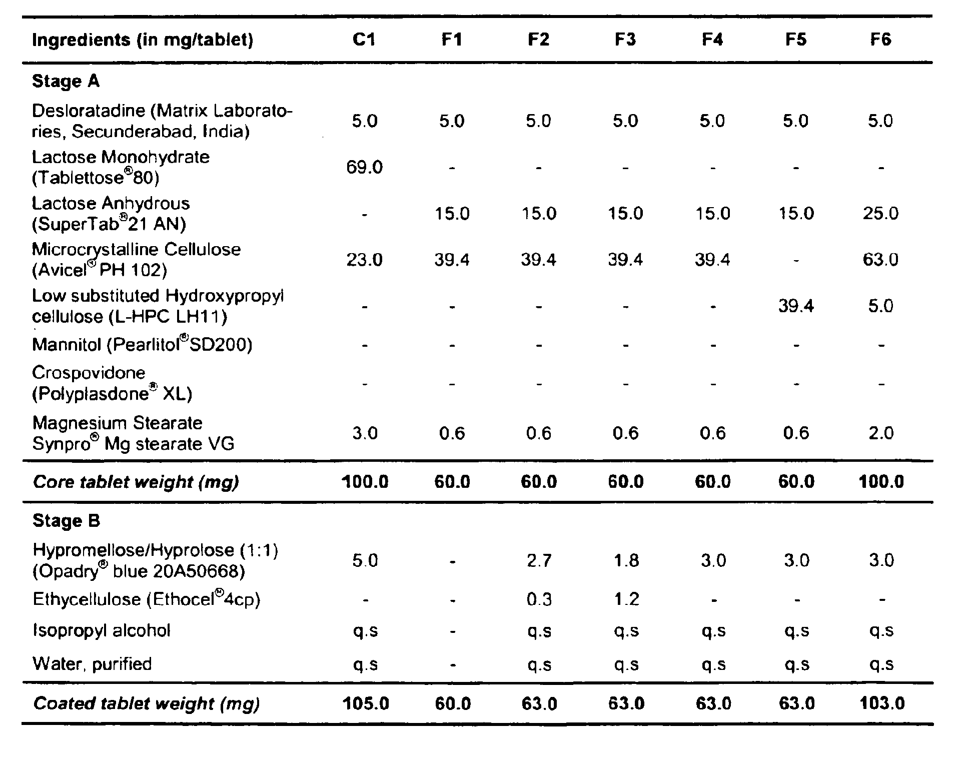

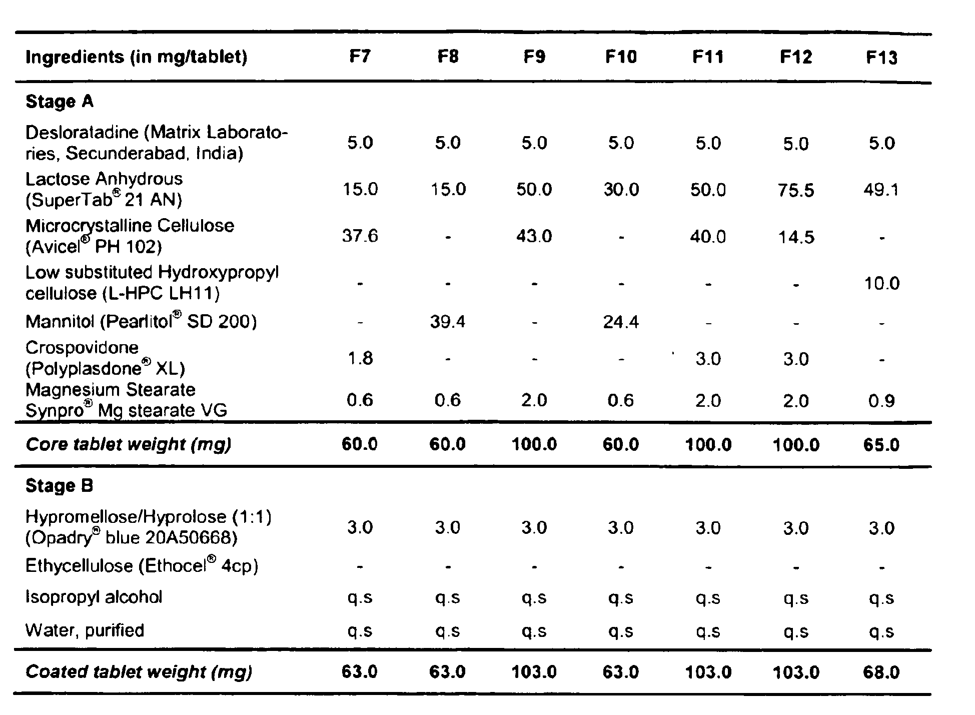

### Procedure I: direct compression (F5 to F8 and F10 to F12):

1. sift all the ingredients of stage A through # 40 mesh (= 0.4 mm),
2. blend the sifted ingredients in a blender (pillar blender, Tapasya Engineering Works Pvt. Ltd.), and
3. compress the tablets using 5.5 mm biconcave, round punches (Ajas Components Pvt. Ltd.).

### Procedure II: dry granulation-compression (F1 to F4, F9 and F13):

1. sift ingredients of stage - A through # 40 mesh (= 0.4 mm),
2. blend all the sifted ingredients in blender (pillar blender, Tapasya Engineering Works Pvt. Ltd.).
3. compact the blend using 16 mm FFBE punch (Ajas Components Pvt. Ltd.) to form slugs,
4. mill the slugs in multi mill using 10 mm screen, knives forward at medium speed (Multimill Lab, Gansons Ltd.),
5. pass the milled slugs through # 30 mesh (= 0.6 mm),
6. mill the retentions in multi mill using 1 mm screen knives forward at high speed (Multimill Lab, Gansons Ltd.),
7. pass the milled granules through # 30 mesh (= 0.6 mm),
8. lubricate the granules with # 40 mesh (= 0.4 mm) pre sifted magnesium stearate, and
9. compress tablets using 5.5 mm s/c tooling (Rotary Tablet Machine CMD3-16, Cadmach Machinery Co. Pvt. Ltd.).

Optionally the core tablets produced by I: direct compression or II: dry granulation are coated as follows:
1. disperse coating material in isopropyl alcohol under stirring,
2. add required quantity of water to step 1,
3. continue stirring up to 45 min, to get homogeneous solution.
4. pass the solution through # 60 mesh (= 0.25 mm), and
5. coat the tablets in automated perforated coating machine (Ganscoater, Gansons Ltd.).

### Part B: Stability of inventive pharmaceutical compositions

The present invention provides solid pharmaceutical compositions in dosage form of a tablet comprising desloratadine and anhydrous lactose, which are improved in their stability compared to compositions comprising desloratadine and lactose monohydrate, whereby the improved stability is shown by reduction of impurities.

Inventive solid pharmaceutical composition in form of coated tablets produced according to formulation F13 in Part A above as well as a comparative composition in form of coated tablets produced according to formulation C1 in Part A above are used to carry out stability tests under conditions of 40°C and 75 % relative humidity (RH) for 1 to 3 months (accelerated storage conditions, ICH guideline Q 1 A (R2)).

The amount of loratadine, unknown impurities (unknown imp.) and total amount of impurities (total imp.) in wt.-% based on the total weight of the coated tablets of the inventive composition F13 and the comparative composition C1 are measured (according to European Pharmacopoeia 6.5, section 5.10) initially after production (in the following initially) and when stored in high density polyethylene containers (in the following HDPE; after storage time of 1, 2 or 3 months (in the following 1 M, 2M or 3M):

| **Formulation** | **Condition** | **Loratadine** | **Unknown Imp.** | **Total Imp.** |
|---|---|---|---|---|
| C1 | Initial | 0.00 | 0.02 | 0.02 |
| | 40°C/75%RH,1M | 0.00 | 0.07 | 0.13 |
| | 40°C/75%RH,2M | 0.00 | 0.12 | 0.19 |
| | 40°C/75%RH,3M | 0.00 | 0.17 | 0.31 |
| F13 | Initial | 0.00 | 0.03 | 0.05 |
| | 40°C/75%RH,1M | 0.00 | 0.05 | 0.09 |
| | 40°C/75%RH,2M | 0.00 | 0.06 | 0.11 |
| | 40°C/75%RH,3M | 0.00 | 0.07 | 0.12 |

Accordingly, the total amount of impurities and the amount of impurities relative to the initial amount for coated tablets of the comparative composition C1 after storage for one, two or three months is higher than for coated tablets of the inventive composition F13 and, thus, inventive compositions comprising desloratadine and anhydrous lactose show an improved stability over compositions of the state of the art comprising desloratadine and lactose monohydrate.

## Claims

1. Solid pharmaceutical composition in the dosage form of a tablet comprising desloratadine or a pharmaceutically acceptable salt thereof and one or more pharmaceutically acceptable excipients and/or adjuvants including lactose, **characterized in that** a) desloratadine or the pharmaceutically acceptable salt thereof is present in a therapeutically effective amount and that b) the lactose is present as anhydrous lactose in an amount of 22 to 78 wt.-% based on the total weight of the solid pharmaceutical composition and that the tablet is obtainable by a process comprising the following steps:
a. providing desloratadine or a pharmaceutically acceptable salt thereof and one or more pharmaceutically acceptable excipients and/or adjuvants including anhydrous lactose,
b. blending the components provided in step a),
c. optional compacting the blended components of step b), and
d. compression of the blended components of step b) or compacts of step c) to form the solid pharmaceutical composition in dosage form of a tablet,
with the proviso, that the process steps a) through d) are carried out under dry conditions.

2. Solid pharmaceutical composition according to claim 1, wherein the further pharmaceutically acceptable excipients and/or adjuvants comprise one or more of the following substances, which act as binders and/or disintegrants: microcrystalline cellulose, maize starch, povidone, hydroxypropyl cellulose, copovidone, pregelatinized starch, dibasic calcium phosphate, sugar, low substituted hydroxypropylcellulose, mannitol, crospovidone, sodium starch glycolate, croscarmellose sodium or mixtures thereof.

3. Solid pharmaceutical composition according to claim 2, wherein the substance or the substances which act as binders and/or disintegrants are selected from the group of a) microcrystalline cellulose, b) low substituted hydroxypropylcellulose, c) mannitol, d) the combination of microcrystalline cellulose and low substituted hydroxypropylcellulose, or e) the combination of microcrystalline cellulose and crospovidone.

4. Solid pharmaceutical composition according to any one of the preceding claims, wherein the further pharmaceutically acceptable excipients and/or adjuvants comprise one or more lubricants, preferably including magnesium stearate.

5. Solid pharmaceutical composition according to claim 4, wherein the amount of the lubricant is in the range of 0.5 to 5 wt.-% based on the total weight of the solid pharmaceutical composition.

6. Solid pharmaceutical composition in dosage form of a tablet according to any one of the preceding claims, wherein the solid pharmaceutical composition in dosage form of a tablet additionally comprises a pharmaceutically acceptable coating.

7. Solid pharmaceutical composition according to claim 6, wherein the pharmaceutically acceptable coating comprises one or more of the following components: hydroxypropyl cellulose, hydroxypropylmethyl cellulose, ethyl cellulose, polyvinyl alcohol, polyethylene glycol-polyvinyl alcohol graftpolymer or mixtures thereof.

8. Solid pharmaceutical composition according to any one of the preceding claims comprising a therapeutically effective amount of one or more additional active ingredients.

9. Solid pharmaceutical composition according to claim 8, wherein the or one of the additional active ingredients is/are present in the coating of the solid pharmaceutical composition in dosage form of a tablet.

10. Process of production of a solid pharmaceutical composition in dosage form of a tablet according to any one of the preceding claims, **characterized in that** the process comprises the following steps:
a. providing desloratadine or a pharmaceutically acceptable salt thereof and one or more pharmaceutically acceptable excipients and/or adjuvants including anhydrous lactose,
b. blending the components provided in step a),
c. optional compacting the blended components of step b), and
d. compression of the blended components of step b) or compacts of step c) to form the solid pharmaceutical composition in dosage form of a tablet **characterized in that** a) desloratadine or the pharmaceutically acceptable salt thereof is present in a therapeutically effective amount and that b) the lactose is present as anhydrous lactose in an amount of 22 to 78 wt.-% based on the total weight of the solid pharmaceutical composition in dosage form of a tablet,
with the proviso, that the process steps a) through d) are carried out under dry conditions.

11. Process according to claim 10, wherein the compaction of step c) is carried out by dry granulation.

12. Process according to claim 10 or 11, wherein the compressed solid pharmaceutical composition of step d) in dosage form of a tablet is in a subsequent step e) coated with one or more pharmaceutically acceptable excipients and/or adjuvants.

13. Use of anhydrous lactose in the production of a solid pharmaceutical composition according to any one of claims 1 to 9, wherein the amount of anhydrous lactose is from 22 to 78 wt.-% based on the total weight of the solid pharmaceutical composition in dosage form of a tablet.

14. Use of a solid pharmaceutical composition according of any one of claims 1 to 9, wherein the solid pharmaceutical composition in dosage form of a tablet is used in the manufacture of a medicament for the treatment or prophylaxis of histamine-induced disorders.

## Patentansprüche

1. Feste pharmazeutische Zusammensetzung in Dosierungsform einer Tablette umfassend Desloratadin oder ein pharmazeutisch akzeptables Salz hiervon und ein oder mehrere pharmazeutische Hilfsstoffe und/oder Zusatzstoffe einschließlich Laktose, **dadurch gekennzeichnet, dass** a) Desloratadin oder ein pharmazeutisch akzeptables Salz hiervon in einer therapeutisch wirksamen Menge vorliegt, und dass b) Laktose als wasserfreie Laktose in einer Menge von 22 bis 78 Gew.-% basierend auf dem Gesamtgewicht der Tablette vorliegt und dass die Tablette erhältlich ist durch ein Verfahren umfassend die folgenden Schritte:
a. bereitstellen von Desloratadin oder eines pharmazeutisch akzeptablen Salzes hiervon und ein oder mehrerer pharmazeutisch akzeptabler Hilfsstoffe und/oder Zusatzstoffe einschließlich Laktose,
b. mischen der in Schritt a) bereitgestellten Komponenten,
c. gegebenenfalls verdichten der in Schritt b) vermischten Komponenten, und
d. komprimieren der in Schritt b) vermischten Komponenten oder der Verdichtungen aus Schritt c) zur Ausbildung einer festen pharmazeutischen Zusammensetzung in Dosierungsform einer Tablette,
mit der Maßgabe, dass die Verfahrensschritte a) bis d) unter trockenen Bedingungen ausgeführt werden.

2. Feste pharmazeutische Zusammensetzung gemäß Anspruch 1, wobei die weiteren pharmazeutisch akzeptablen Hilfsstoffe und/oder Zusatzstoffe ein oder mehrere der folgenden Substanzen umfassen, die als Bindemittel oder Sprengmittel agieren: Mikrokristalline Cellulose, Maisstärke, Povidon, Hydroxypropylcellulose, Mannitol, Crospovidon, Natriumstärkeglykolat, Croscaramellose-Natrium oder Mischungen hiervon.

3. Feste pharmazeutische Zusammensetzung gemäß Anspruch 2, wobei die Substanz oder die Substanzen, die als Bindemittel und/oder Sprengmittel agieren, ausgewählt werden aus der Gruppe von a) Mikrokristalliner Cellulose, b) niedrig substituierter Hydroxypropylcellulose, c) Mannitol, d) der Kombination von Mikrokristalliner Cellulose und niedrig substituierter Hydroxypropylcellulose oder a) der Kombination von Mikrokristalliner Cellulose und Crospovidon.

4. Feste pharmazeutische Zusammensetzung gemäß einem der vorstehenden Ansprüche, wobei die weiteren pharmazeutisch akzeptablen Hilfsstoffe und/oder Zusatzstoffe ein oder mehrere Schmiermittel umfassen, vorzugsweise einschließlich Magnesiumstearat.

5. Feste pharmazeutische Zusammensetzung gemäß Anspruch 4, wobei die Menge des Schmiermittels im Bereich 0,5 bis 5 Gew.-% bezogen auf das Gesamtgewicht der festen pharmazeutischen Zusammensetzung beträgt.

6. Feste pharmazeutische Zusammensetzung in Dosierungsform einer Tablette gemäß einem der vorstehenden Ansprüche, wobei die feste pharmazeutische Zusammensetzung in Dosierungsform einer Tablette zusätzlich eine pharmazeutisch akzeptable Beschichtung umfasst.

7. Feste pharmazeutische Zusammensetzung gemäß Anspruch 6, wobei die pharmazeutische akzeptable Beschichtung ein oder mehrere der folgenden Komponenten umfasst: Hydroxypropylcellulose, Hydroxypropylmethylcellulose, Ethylcellulose, Polyvinylalkohol, Polyethylenglykol-Polyvinylalkohol-Propfpolymer oder Mischungen hiervon.

8. Feste pharmazeutische Zusammensetzung gemäß einem der vorstehenden Ansprüche umfassend eine therapeutisch wirksame Menge eines oder mehrerer zusätzlicher aktiver Inhaltsstoffe.

9. Feste pharmazeutische Zusammensetzung gemäß Anspruch 8, wobei die oder einer der zusätzlichen aktiven Inhaltsstoffe in der Beschichtung der festen pharmazeutischen Zusammensetzung in Dosierungsform einer Tablette vorliegen.

10. Verfahren zur Herstellung einer festen pharmazeutischen Zusammensetzung in Dosierungsform einer Tablette gemäß einem der vorstehenden Ansprüche, **dadurch gekennzeichnet, dass** das Verfahren folgende Schritte umfasst:
a. bereitstellen von Desloratadin oder eines pharmazeutisch akzeptablen Salzes hiervon und ein oder mehrere pharmazeutisch akzeptable Hilfsstoffe und/oder Zusatzstoffe einschließlich wasserfreier Laktose,
b. mischen der in Schritt a) bereitgestellten Komponenten,
c. gegebenenfalls verdichten der in Schritt b) vermischten Komponenten, und
d. komprimieren der in Schritt b) vermischten Komponenten oder der Verdichtungen aus Schritt c) zur Ausbildung einer festen pharmazeutischen Zusammensetzung in Dosierungsform einer Tablette, **dadurch gekennzeichnet, dass** a) Desloratadin oder ein pharmazeutisch akzeptables Salz hiervon in einer therapeutisch wirksamen Menge vorliegt, und dass b) Laktose als wasserfreie Laktose in einer Menge von 22 bis 78 Gew.-% basierend auf dem Gesamtgewicht der Tablette vorliegt,
mit der Maßgabe, dass die Verfahrensschritte a) bis d) unter trockenen Bedingungen ausgeführt werden.

11. Verfahren gemäß Anspruch 10, wobei die Verdichtung in Schritt c) mittels Trockengranulation ausgeführt wird.

12. Verfahren gemäß Anspruch 10 oder 11, wobei die komprimierte feste pharmazeutische Zusammensetzung aus Schritt d) in Dosierungsform einer Tablette in einem nachfolgenden Schritt e) mit ein oder mehreren pharmazeutische akzeptablen Hilfsstoffen und/oder Zusatzstoffen beschichtet wird.

13. Verwendung von wasserfreier Laktose in der Herstellung einer festen pharmazeutischen Zusammensetzung gemäß einem der Ansprüche 1 bis 9, wobei wasserfreie Laktose in einer Menge von 22 bis 78 Gew.-% basierend auf dem Gesamtgewischt der festen pharmazeutischen Zusammensetzung in Dosierungsform einer Tablette vorliegt.

14. Verwendung einer festen pharmazeutischen Zusammensetzung gemäß einem der Ansprüche 1 bis 9, wobei die feste pharmazeutische Zusammensetzung in Dosierungsform einer Tablette in der Herstellung eines Arzneimittels zur Behandlung oder Prophylaxe von Histamin-induzierten Erkrankungen verwendet wird.

## Revendications

1. Composition solide pharmaceutique sous la forme de dosage d'un tablette comprenant du desloratadine ou un sel pharmaceutiquement acceptable et un ou plusieurs excipients et/ou adjuvants pharmaceutiquement acceptable inclus du lactose, **caractérisé en ce que** a) la desloratadine ou un sel pharmaceutiquement acceptable soit présent en quantité thérapeutique efficace et que b) le lactose soit présent sous la forme de lactose anhydre dans une quantité de 22 à 78 wt. % par rapport au poids total de la composition pharmaceutique totale et que la tablette susceptible obtenu par le procédé comprenant les étapes suivantes :
a. tenir prêt de desloratadine ou d'un sel pharmaceutiquement acceptable et un ou plusieurs excipients et/ou adjuvants pharmaceutiquement acceptable inclus du lactose anhydre,
b. mélanger des composants, lequelles on a tenu prêt en l'étape a),
c. compacter optionnelle du mélange de composants de l'étape b), et
d. comprimer du mélange de composants de l'étape b) ou du mélange compacté de l'étape c) pour former la composition pharmaceutique solide sous la forme de dosage d'une tablette,
sous la condition que les étapes a) à d) du procédé soient exécutée dans des conditions sèches.

2. Composition solide pharmaceutique selon la revendication 1, dans laquelle les excipients et/ ou adjuvants pharmaceutiquement acceptable comprenant une ou plusieurs des substances suivantes, qui agissent comme liants et/ou désintégrants : cellulose de microcrystalline, amidon de maïs, povidone, cellulose d'hydroxypropyl, copovidone, amidon de prégélatiné, phosphate de calcium dibasique, sucre, cellulose d'hydroxypropyl substituée basse, mannitol, crospovidone, glycolate d'amidon de sodium, sodium de croscarmellose ou mélanges dérivés.

3. Composition solide pharmaceutique selon la revendication 2, où la ou les substances qui agissent comme liants et /ou comme désintégrants sont choisis parmi le groupe de a) cellulose de microcrystalline, b) cellulose d'hydroxypropyl substituée basse, c) mannitol, d) la combinaison de cellulose de microcrystalline et de cellulose d'hydroxypropyl substituée basse, et e) la combinaison de cellulose de microcrystalline et de crospovidone.

4. Composition solide pharmaceutique selon l'une quelconque des revendications précédentes dans laquelle les excipients suivants et / ou adjuvants pharmaceutiquement acceptable comprenant un ou plusieurs lubrifiants, incluant de préférence du stéarate de magnésium.

5. Composition solide pharmaceutique selon la revendication 4 dans laquelle la quantité de lubrifiant va de 0,5 à 5 wt % par rapport au poids total de la composition pharmaceutique solide.

6. Composition solide pharmaceutique sous la forme de dosage d'une tablette selon l'une quelconque des revendications précédentes, dans lesquelles la composition pharmaceutique solide sous la forme de dosage d'une tablette comprend par ailleurs un enrobage pharmaceutiquement acceptable.

7. Composition solide pharmaceutique selon la revendication 6 dans laquelle l'enrobage pharmaceutiquement acceptable comprenant un ou plusieurs des composants suivants : cellulose d'hydroxypropyl, cellulose ethylique, alcool polyvinyl, polymère de greffe d'alcool de glycopolyvinyl de polyethylene, ou mélange de ceux-ci.

8. Composition solide pharmaceutique selon l'une quelconque des revendications précédentes comprenant une quantité efficace de façon thérapeutique de l'un ou de plusieurs ingrédients actifs additionnels.

9. Composition solide pharmaceutique selon la revendication 8 dans laquelle les ou l'un des ingrédients actifs additionnels est/sont présent dans l'enrobage de la composition solide pharmaceutique sous la forme de dosage d'une tablette.

10. Procédé pour la production de composition pharmaceutique solide sous la forme de dosage d'une tablette selon l'une quelconque des revendications précédentes, **caractérisé en ce que** le procédé comprenant les étapes suivantes :
a. tenir prêt de desloratadine ou d'un sel pharmaceutiquement acceptable et un ou plusieurs excipients et/ou adjuvants pharmaceutiquement acceptable inclus du lactose anhydre,
b. mélanger des composants, lesquelles on a tenu prêt en l'étape a),
c. compacter optionnelle du mélange de composants de l'étape b), et
d. comprimer du mélange de composants de l'étape b) ou du mélange compacté de l'étape c) pour former la composition pharmaceutique solide sous la forme de dosage d'une tablette **caractérisée par le fait que** a) la desloratadine ou le sel pharmaceutiquement acceptable est présent dans une quantité efficace de façon thérapeutique et que b) le lactose est présent sous forme de lactose anhydre dans une quantité de 22 à 78 wt % du poids total de la composition pharmaceutique solide sous la forme de dosage d'une tablette,
sous la condition que les étapes a) à d) du procédé soient exécutée dans des conditions sèches.

11. Procédé selon la revendication 10 dans lequel le compactage de l'étape c) est exécuté par granulation sèche.

12. Procédé selon la revendication 10 ou 11 dans lequel la composition pharmaceutique solide de l'étape d) sous la forme de dosage de tablette est enrobée au cours d'une étape e) ultérieure avec un ou plusieurs excipients et / ou des adjuvants pharmaceutiquement acceptable.

13. Utilisation de lactose anhydre dans la production d'une composition pharmaceutique solide selon l'une quelconque des revendications 1 à 9, dans lesquelles la quantité de lactose anhydre doit être de 22 à 78 wt % du poids total de la composition solide pharmaceutique sous la forme de dosage d'une tablette.

14. Utilisation d'une composition solide pharmaceutique selon l'une quelconque des revendications dans lesquelles la composition solide pharmaceutique sous la forme de dosage d'une tablette est utilisée pour la fabrication d'un médicament pour le traitement ou la prophylaxis des maladies provoqué par l'histamine.
